# EUROPÄISCHE PATENTSCHRIFT

(11) **EP 2 044 900 B1**
(45) Veröffentlichungstag und Bekanntmachung des Hinweises auf die Patenterteilung: **21.04.2010**
(21) Anmeldenummer: 09000950.7
(22) Anmeldetag: 27.04.2005
(51) Int. Cl.: A61B 18/14

(54) **Elektrochirurgisches Instrument**
Electrosurgical instrument
Instrument d'électrochirurgie

(30) Priorität: 14.05.2004 DE 102004024052; 28.05.2004 DE 102004026179
(43) Veröffentlichungstag der Anmeldung: 08.04.2009
(62) Teilanmeldung aus: 05744806.0
(73) Patentinhaber: Erbe Elektromedizin GmbH, 72072 Tübingen (DE)
(72) Erfinder: Fischer, Klaus, 72202 Nagold (DE)
(74) Vertreter: Bohnenberger, Johannes

(56) Entgegenhaltungen:
- EP-A- 0 253 012
- WO-A-03/099372
- WO-A-2004/032777
- DE-A1- 19 730 724
- US-A- 4 498 475
- US-A- 6 059 782
- US-A1- 2001 037 109
- US-A1- 2002 068 951
- US-B1- 6 267 761
- US-B1- 6 270 497

## Beschreibung

Die Erfindung betrifft ein elektrochirurgisches Instrument nach dem Oberbegriff des Patentanspruches 1.

Elektrochirurgische Instrumente werden seit vielen Jahren in der Hochfrequenz-Chirurgie eingesetzt, um biologisches Gewebe zu koagulieren und/oder zu schneiden. Dabei wird ein hochfrequenter Strom durch das zu behandelnde Gewebe geleitet, so dass sich dieses aufgrund Eiweißkoagulation und Dehydratation verändert. Das Gewebe zieht sich dabei derart zusammen, dass die Gefäße verschlossen und Blutungen gestillt werden. Eine darauf folgende Erhöhung der Stromdichte bewirkt ein explosionsartiges Verdampfen der Gewebeflüssigkeit und ein Aufreißen der Zellmembranen, wobei das Gewebe vollständig durchtrennt wird. Verfahren dieser Art weisen gegenüber einem rein mechanisch vorgenommenen Schnitt den Vorteil einer Hämostase der Schnittränder auf.

Der Einsatz bipolarer Instrumente gewinnt immer mehr an Bedeutung, da geringere Stromstärken als bei monopolaren Instrumenten nötig sind. Von Vorteil ist es insbesondere, dass der Stromweg zwischen den Elektrodenteilen bipolarer Instrumente kalkulierbar ist und nicht weite Strecken durch den Körper des Patienten verläuft.

Bipolare Instrumente weisen im Wesentlichen zwei gelenkig miteinander verbundene Klemmenteile auf, wobei an deren proximalen Enden Griffeinrichtungen zur Handhabung der Klemmenteile vorgesehen sind. An distalen Enden der Klemmenteile befinden sich Elektrodenteile zum Fassen von Gewebe und zum Durchleiten eines Koagulationsstromes durch das Gewebe. Der von einem HF-Generator erzeugte HF-Strom wird über Stromzuführungseinrichtungen zu den Elektrodenteilen des bipolaren Instrumentes geleitet.

Bei Verwendung oben beschriebener bipolarer Instrumente müssen nach einem Koagulationsvorgang Schneidinstrumente eingesetzt werden, um das koagulierte Gewebe nun endgültig zu durchtrennen. Der Schnitt wird entweder mit einer chirurgischen Schere oder einem HF-Schneidinstrument durchgeführt. Der Einsatz verschiedener Instrumente erfordert jedoch eine Unterbrechung des chirurgischen Eingriffs und zögert diesen unnötig hinaus.

Um diesem Nachteil zu begegnen, werden mittlerweile multifunktionale Instrumente eingesetzt, die zumindest für das Koagulieren und für das Schneiden ausgelegt sind. Ein derartiges Instrument ist beispielsweise aus der DE 199 15 060 A1 bekannt, bei der diverse Arbeitseinrichtungen, wie z. B. Zangen, Haken oder auch Ultraschalleinrichtungen und Elektroden zum Schneiden oder Koagulieren über Aktoren betätigbar sind. Eine Steuerungseinheit ermöglicht das sukzessive Abarbeiten vorgesehener Arbeitsschritte.

Die hier beschriebene Ausführungsform eines multifunktionalen Instrumentes hat jedoch den Nachteil, dass Koagulieren und Schneiden nach wie vor zwei verschiedene, zeitlich hintereinander auszuführende Handlungen sind, auch wenn die Schritte durch ein einziges Instrument ausgeführt werden können. Ein erster Vorgang muss demnach bewusst beendet und ein zweiter Vorgang auch wieder bewusst begonnen werden. Zwischen den Vorgängen ist mindestens eine Handlung auszuführen, nämlich das multifunktionale Instrument für die nächste Aufgabe zu aktivieren. Auch dies verzögert unnötig den Operationsverlauf. Zudem können Fehler bei der Aktivierung eines Vorganges hinsichtlich der Einstellung entsprechender Betriebsparameter, wie z. B. eines geeigneten HF-Stromes auftreten.

Bekannte Multifunktionsinstrumente der oben beschriebenen Art weisen außerdem elektrisch voneinander isolierte, jeweils für das Koagulieren und für das Schneiden vorgesehene Pole auf, so dass sich für das Instrument relativ große Abmessungen ergeben. Dies schränkt die Bewegungsfreiheit des Chirurgen am Operationsgebiet deutlich ein und begrenzt somit das Einsatzgebiet der bekannten Instrumente.

Der Erfindung liegt daher die Aufgabe zu Grunde, ein elektrochirurgisches Instrument der eingangs genannten Art dahin gehend weiterzubilden, dass eine insbesondere mehrere Verfahrensgänge benötigende Operation auf einfachste Art und mit optimiertem Ablauf durchführbar ist.

Dokument US-B-6270497 offenbart ein Instrument gemäß Oberbegriff des Anspruchs 1.

Diese Aufgabe wird durch ein elektrochirurgisches Instrument nach Patentanspruch 1 gelöst.

Insbesondere wird die Aufgabe durch ein elektrochirurgisches Instrument gelöst, das zwei gelenkig miteinander verbundenen Branchen umfasst, die zum Öffnen oder Schließen entsprechend einem Klemm-, Spreiz- oder Schneidwerkzeug betätigbar sind. Ferner umfasst das Instrument Elektrodenteile an distalen Enden der Branchen zum Fassen von Gewebe und zum Durchleiten eines Koagulationsstromes durch das Gewebe zu dessen Koagulation, die elektrisch voneinander isoliert sind sowie Stromzuführungseinrichtungen zum Zuführen des Koagulationsstromes zu den Elektrodenteilen. An dem Instrument ist ferner mindestens an einem Elektrodenteil ein als Schneidelektrode ausgelegter Schneidabschnitt ausgebildet, so dass das Elektrodenteil den Schneidabschnitt und einen Koagulationsabschnitt aufweist. Zudem ist eine Steuerungseinheit zur Steuerung des HF-Stromes derart vorgesehen, dass bei Erreichen eines Eigenschaften des erfassten Gewebes kennzeichnenden Schwellenwertes mindestens dem Schneidabschnitt ein Schneidstrom zugeführt wird, der unterschiedlich ist zum Koagulationsstrom.

Ein wesentlicher Punkt der Erfindung liegt darin, dass ein Schneidabschnitt derart an einem für die Koagulation ausgelegten Elektrodenteil angeordnet ist, dass er zu einem geeigneten Zeitpunkt, d. h. bei Erreichen eines bestimmten Operationsstadiums als Schneidelektrode wirkt. Der Chirurg ist daher beim Übergang von einer Koagulationsphase in eine nachfolgende Schneidphase nicht mit Entscheidungsaufgaben belastet. Gleichzeitig sind optimale Betriebsparameter, wie z. B. die richtige Stromstärke, generierbar, ohne dass der Operateur diese am HF-Generator über die Spannung selbsttätig einstellen muss. So sind der zeitliche Ablauf zwischen den einzelnen Operationsphasen und der dafür benötigte HF-Strom optimal aufeinander abgestimmt.

Der Eingriff ist daher unter weitestgehender Ausschaltung von Fehlerquellen durchführbar.

Bevorzugte Weiterbildungen der Erfindung sind in den Unteransprüchen angegeben.

In einer ersten bevorzugten Ausführungsform sind der Steuerungseinheit Schalteinrichtungen zugeordnet, die den Schwellenwert als definierten Abstand zwischen den Branchen erfassen, so dass der Schneidstrom in Abhängigkeit des Abstandes zugeführt wird. Sobald ein vorgegebener Abstand zwischen den Branchen, d. h. zwischen den Elektrodenteilen unterschritten wird, erfolgt eine Zufuhr des Schneidstromes mindestens an den Schneidabschnitt. Die Schalteinrichtungen leiten dazu bei Betätigung ein Signal an die Steuerungseinheit weiter, wobei diese über einen Hochfrequenz-Generator veranlasst, dass dem Schneidabschnitt ein entsprechender Schneidstrom zugeführt wird. Der Abstand dient als Kennzeichen dafür, dass ein Schneidvorgang durchgeführt werden kann, d. h., dass die Elektrodenteile einen Abstand zueinander aufweisen, bei dem ein Schneiden überhaupt erst möglich ist. Der Abstand zwischen den Elektrodenteilen ist dabei über die Höhe der eingestellten HF-Spannung definierbar.

Die Schalteinrichtungen sind vorteilhafterweise an mindestens einer der Branchen und/oder an einem mindestens an einer der Branchen angeordneten Abstandshalter vorgesehen. Dies ist deshalb vorteilhaft, weil der Abstand dann unmittelbar von den Schalteinrichtungen erfasst und von diesen gleichzeitig der Schneidvorgang ausgelöst werden kann.

In einer bevorzugten Ausführungsform sind die Schalteinrichtungen als ein Tastschalter ausgebildet. Dieser ist dann vorzugsweise an dem Abstandshalter der einen Branche angebracht, so dass bei Berührung des Schalters durch die gegenüberliegende Branche und damit bei Erreichen des Schwellenwertes - hier dem definierten Abstand zwischen den Elektrodenteilen - der Schneidstrom dem Schneidabschnitt zugeführt wird. Dies ist eine besonders einfache Ausgestaltung, um auf kostengünstige Weise bei Unterschreiten des bestimmten Abstandes zwischen den Elektrodenteilen den Schneidvorgang auszulösen.

In einer weiteren bevorzugten Ausführungsform sind die Schalteinrichtungen als ein berührungsloser Schalter ausgebildet. Diese haben den Vorteil, dass keine Berührung der Branchen vorgesehen werden muss und der Mechanismus daher weniger verschleißanfällig ist und präzise arbeitet.

Vorteilhafterweise sind die berührungslosen Schalter dann beispielsweise als Näherungsschalter oder auch als Reedkontakt ausgebildet. Ist z. B. ein Reedkontakt an der einen Branche angebracht und ein Magnet an der gegenüberliegenden Branche, so schaltet der Reedkontakt, sobald der Magnet zu dem Reedkontakt einen bestimmten Abstand aufweist. Ähnlich arbeitet ein Näherungsschalter, beispielsweise ein induktiver Näherungsschalter. Der an der einen Branche angebrachte Näherungsschalter schaltet, sobald ein an der gegenüberliegenden Branche angeordneter Metallgegenstand Wirbelströme in einem elektromagnetischen Wechselfeld des Näherungsschalters erzeugt. Bei einem Schalter der hier beschriebenen Art kann der Schaltabstand vorzugsweise mitunter durch den in das Wechselfeld eingebrachten Metallgegenstand definiert werden.

Vorteilhafterweise ist der Steuerungseinheit eine Einrichtung zur Widerstandsmessung zugeordnet, die den Schwellenwert als ohm'schen Widerstand des Gewebes erfasst, so dass der Schneidstrom in Abhängigkeit des ohm'schen Widerstandes zugeführt wird. Die Widerstandsmessung des Gewebes erlaubt die Ermittlung eines präzisen Zeitpunktes, ab wann ein Schneidvorgang gestartet werden kann. Sobald in dem Gewebe aufgrund des Operationsverlaufes ein definierter Widerstand erreicht ist, veranlasst die Steuerungseinheit, dass dem Schneidabschnitt der entsprechenden Schneidstrom zugeführt wird. Dieses Verfahren ist äußerst zuverlässig, weil der durch die Koagulation veränderte Gewebewiderstand einen präzisen Anhaltspunkt dafür gibt, wann mit einem Schneidvorgang begonnen werden kann.

In einer weiteren bevorzugten Ausführungsform ist der Steuerungseinheit ein Lichtbogenmonitor und/oder Strommonitor zugeordnet, die den Schwellenwert als optimalen Koagulationsendzeitpunkt erfassen, so dass der Schneidstrom in Abhängigkeit des Koagulationsendzeitpunktes zugeführt wird. Das heißt, der Schneidstrom wird mindestens dem Schneidabschnitt zugeführt, sobald die Koagulation aufgrund des von dem entsprechenden Monitor gelieferten Signals beendet wird. Damit erfolgt die Zuführung des Schneidstromes vorteilhafterweise zu einem für den Operationsverlauf idealen Zeitpunkt. Die Funktionsweisen des Strommonitors und Lichtbogenmonitors sind beispielsweise in der EP 0 253 012 B1 ausführlich beschrieben.

Vorzugsweise ist der Schneidabschnitt an dem mindestens einen Elektrodenteil als ein in Bezug auf den Koagulationsabschnitt des mindestens einen Elektrodenteils verjüngter Bereich ausgebildet. Dabei können Koagulationsabschnitt und Schneidabschnitt eine integral ausgebildete Elektrode ausgestalten oder aber die beiden Bereiche sind unabhängig voneinander angeordnet. Die verjüngte Ausbildung des Schneidabschnittes ermöglicht eine für das Schneiden von Gewebe erforderliche Erhöhung der Stromdichte an dem Schneidabschnitt. Das den Koagulationsabschnitt und den Schneidabschnitt integral ausbildende Elektrodenteil kann während eines Koagulationsvorganges über ihren gesamten Flächenbereich, d. h. sowohl über den Flächenbereich des Koagulationsabschnittes als auch über den Flächenbereich des Schneidabschnittes, als Koagulationselektrode wirken, während der verjüngt ausgebildete Schneidabschnitt allein für einen späteren Schneidvorgang zur Verfügung steht. Bei getrennt voneinander ausgebildeten Bereichen zum Koagulieren und Schneiden sind diese sowohl in Kombination als auch getrennt voneinander einsetzbar. Dem kleineren, als Schneidabschnitt ausgebildeten Flächenbereich wird entsprechend obiger Ausführungen ein adäquater Schneidstrom über die Stromzuführungseinrichtungen zugeführt. Somit ist ein und dasselbe Instrument sowohl zum Koagulieren als auch zum Schneiden zu verwenden.

In einer bevorzugten Ausführungsform ist der Schneidabschnitt als Kante mit einem im Wesentlichen dreieckförmigen Querschnitt an dem mindestens einen Elektrodenteil ausgebildet. Ein dreieckförmiger Querschnitt erlaubt den sukzessiven Übergang von einem großen Flächenbereich des Elektrodenteils bis zu deren kantenförmiger Verjüngung. Der sanfte Übergang ist in besonderem Maße geeignet, das gesamte Elektrodenteil bei ausreichender Gewebedicke als Koagulationselektrode einzusetzen und in einem fortgeschrittenen Operationsstadium den Schneidabschnitt allein zum Schneiden zu verwenden.

Vorteilhafterweise ist der Schneidabschnitt als Kante mit einem im Wesentlichen kreisförmigen Querschnitt an dem mindestens einen Elektrodenteil ausgebildet. Bei dieser Ausführungsform steht eine relative große Elektrodenfläche für den Koagulationsvorgang zur Verfügung, während der als Kante ausgebildete Schneidabschnitt bei genügend großer Gewebedicke kaum ins Gewicht fällt. In einem fortgeschrittenen Operationsstadium hingegen und bei ausreichender Nähe gegenüberliegender Elektrodenteile des elektrochirurgischen Instruments lässt sich aufgrund der kantenförmigen Ausgestaltung des Schneidabschnittes die Stromdichte derart erhöhen, dass ein Schneidvorgang ermöglicht wird.

Eine weitere Lösung sieht vor, den Schneidabschnitt im Wesentlichen kugelförmig an dem mindestens einen Elektrodenteil auszubilden. Damit lässt sich die Schneidefläche größer halten und ein entsprechend breiter Schnitt durchführen.

Die erfindungsgemäße Lösung sieht vor, dass der Schneidabschnitt spitzen-, nadel- oder schlingenförmig ausgebildet ist. Dies entspricht weiteren üblichen Formen von Schneidelektroden, so dass das Schneiden mit den gewohnten Instrumenten für den jeweiligen Anwendungsfall durchführbar ist.

In einer bevorzugten Ausführungsform ist der Schneidabschnitt jeweils an sich gegenüberliegenden Elektrodenteilen ausgebildet. Aufgrund der für das Schneiden ausgelegten Abschnitte ist hier eine besonders präzise Schneidwirkung erreichbar, weil die Stromdichte an beiden Elektrodenteilen erhöht werden kann.

Der Schneidabschnitt kann aber auch außerhalb des Koagulationsabschnittes angeordnet, d. h. getrennt von diesem ausgebildet sein, wie bereits oben erwähnt. Vorteilhafterweise ist der Schneidabschnitt dann mittels Positionierungseinrichtungen als relativ zu dem Koagulationsabschnitt bewegbarer Bestandteil an diesem ausgebildet. Der Schneidabschnitt kann dann während eines Koagulationsvorganges aus dem Koagulationsgebiet entfernt werden, so dass hier keine unerwünschten Schneidwirkungen auftreten. Bei Erreichen des Schwellenwertes lässt sich der Schneidabschnitt bzw. lassen sich die Schneidabschnitte in die entsprechende, für den Schneidvorgang erforderliche Position bringen.

Erfindungsgemäß weisen die Positionierungseinrichtungen einen drehbar in einer der Branchen gelagerten zweiarmigen Hebel mit einem ersten Ende und einem zweiten Ende auf, wobei das erste Ende zur Aufnahme des Schneidabschnittes und das zweite Ende zur Kontaktierung mit der gegenüberliegenden Branche oder einem an der gegenüberliegenden Branche vorgesehenen Abstandshalter vorgesehen ist. Bei Kontaktierung des zweiten Endes mit der gegenüberliegenden Branche ist der Schneidabschnitt in Richtung des gegenüberliegenden Elektrodenteils bewegbar. Die Positionierungseinrichtungen weisen zudem eine Rückstellvorrichtung auf, so dass der Schneidabschnitt nach Beendigung der Kontaktierung zurück in die Ausgangslage bewegbar ist. Somit lässt sich auf besonders einfache Weise der Schneidabschnitt während der Koagulationsphase innerhalb des Koagulationsabschnittes versenken, so dass hier eine Beeinträchtigung des Koagulationsvorganges durch den Schneidabschnitt vermieden wird. Sobald in diesem Falle der Schwellenwert erreicht ist und eine Kontaktierung stattfindet, dreht sich der Schneidabschnitt aus seiner Ruhelage. Eine Zuführung des Schneidstromes an den nun offengelegten Schneidabschnitt kann beispielsweise über die Schalteinrichtungen erfolgen, die entprechend obiger Ausführungen an dem Abstandshalter oder an dem zweiten Ende des Hebels angeordnet sind und ein entsprechendes Signal an die Steuerungseinheit liefern. Aufgrund der einfachen mechanischen Konstruktion ist ein derartiges multifunktionales elektrochirurgisches Instrument preiswert und einfach herstellbar. Die Positionierung des Schneidabschnittes ist zudem unabhängig von einer Handkraft des Chirurgen, weil die Steuerungseinheit lediglich aufgrund der Kontaktierung aktiviert wird. Im Übrigen lässt sich eine soeben beschriebene Positionierungseinrichtung auch an beiden Branchen vorsehen.

Eine bevorzugte Ausführungsform sieht vor, dass am ersten Ende des zweiarmigen Hebels eine Aufnahmevorrichtung für den Schneidabschnitt angebracht ist. Vorteilhafterweise lässt sich der Schneidabschnitt dann temporär entfernen. Dies ist von Vorteil, wenn der Schneidabschnitt nach einer Operationsphase gereinigt werden soll bzw. wenn ein Schneidvorgang nicht vorgesehen ist. Damit lässt sich der Schneidabschnitt auf einfachste Weise deaktivieren.

Eine mögliche Realisierung der Vorrichtung besteht darin, den Schneidabschnitt als integralen Bestandteil des ersten Endes des zweiarmigen Hebels auszubilden. Somit lässt sich die Positionierungseinrichtung auf besonders einfache Weise herstellen.

Eine bevorzugte Ausführungsform sieht vor, dass die Rückstellvorrichtung für den Hebelarm als in der die Positionierungseinrichtungen aufweisenden Branche angeordnetes Federelement vorgesehen ist. Ein Federelement ist ein einfach einzubauendes und kostengünstiges Bauteil, das kaum verschleißanfällig stets die geforderte Funktion erfüllt.

Eine erfindungsgemäße Lösung sieht vor, den Schneidabschnitt aus einer Anti-Haftbeschichtung und/oder aus abbrandfestem Material auszubilden. Der in das zu behandelnde Gewebe eingebrachte HF-Strom verursacht aufgrund der Wärmeentwicklung nicht nur die gewünschten Koagulations- oder Schneideffekte. Vielmehr können z. B. Gewebereste und Blut insbesondere an den Elektrodenteilen der Klemmen so stark anbrennen, dass der Stromfluss beeinträchtigt wird. Eine Anti-Haftbeschichtung reduziert eine derartige Verschmutzung und sollte insbesondere an dem jeweiligen Schneidabschnitt vorgesehen werden. Eine Schicht aus abbrandfestem Material kann den Schneidabschnitt zudem vor Verschleiß aufgrund des hohen HF-Strom schützen.

Nachfolgend wird die Erfindung anhand von Ausführungsbeispielen beschrieben, die anhand der Abbildungen näher erläutert werden. Hierbei zeigen
- - Fig. 1: eine Elektrodenanordnung in einer ersten Ausführungsform;
- - Fig. 2: eine Elektrodenanordnung in einer zweiten Ausführungsform;
- - Fig. 3: eine Elektrodenanordnung in einer dritten Ausführungsform;
- - Fig. 4: eine Elektrodenanordnung in einer vierten Ausführungsform;
- - Fig. 5: ein funktionales Blockschaltbild;
- - Fig. 6: eine Schnittansicht entlang der Linie VI - VI aus Fig. 7 einer Elektrodenanordnung in einer fünften Ausführungsform;
- - Fig. 7: eine Seitenansicht der Elektrodenanordnung gemäß Fig. 6;
- - Fig. 8a: ein Strom-Zeit-Diagramm, in dem der zeitliche Verlauf der Stromstärke bei unterschiedlichen Betriebsmodi dargestellt ist;
- - Fig. 8b: ein Spannungs-Zeit-Diagramm, in dem der zeitliche Verlauf der Spannung bei unterschiedlichen Betriebsmodi gemäß Fig. 8a dargestellt ist.

In der nachfolgenden Beschreibung werden für gleiche und gleich wirkende Teile dieselben Bezugsziffern verwendet.

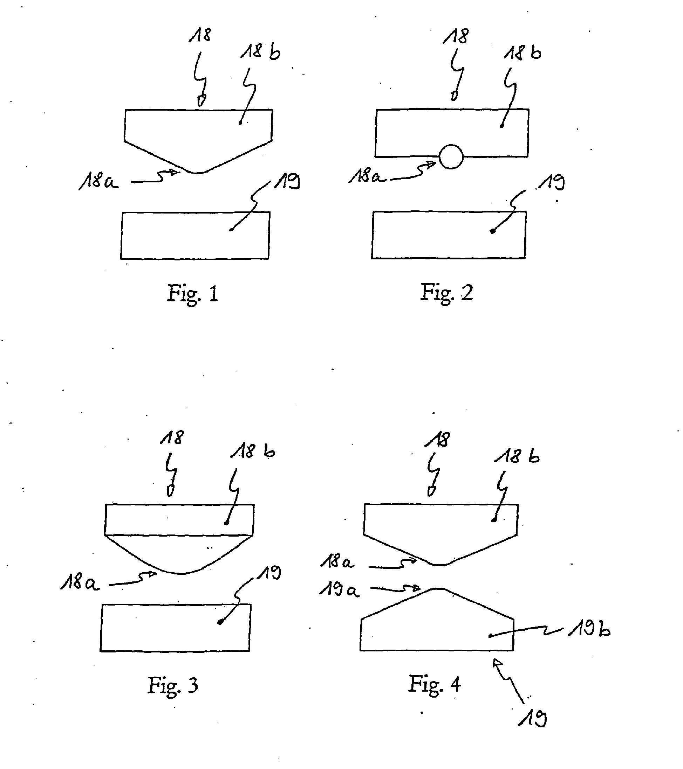

Fig. 1 bis 3 zeigen verschiedene Ausführungsform einer Anordnung von sich gegenüberliegenden Elektrodenteilen 18, 19. Dabei ist jeweils nur an einem Elektrodenteil 18 ein expliziter Schneidabschnitt 18a ausgebildet. Es sei darauf hingewiesen, dass es sich hierbei um schematische Abbildungen handelt, die eine Vorderansicht lediglich der Elektrodenteile 18, 19 zeigen. Das die Elektrodenteile 18, 19 aufnehmende elektrochirurgische Instrument 10 ist nicht dargestellt.

Die Ausbildung des Schneidabschnittes 18a ist idealerweise über eine Verjüngung des jeweiligen Elektrodenteils 18 erreichbar, so dass das Elektrodenteil 18 letztendlich einen Koagulationsabschnitt 18b und einen Schneidbereich, also den Schneidabschnitt 18a aufweist. Die Reduzierung der Elektrodenfläche erlaubt eine für das Schneiden von Gewebe erforderliche Erhöhung der Stromdichte an dem Schneidabschnitt 18a. Das den Koagulationsabschnitt 18b und den Schneidabschnitt 18a integral ausbildende Elektrodenteil 18 kann während eines Koagulationsvorganges über den gesamten Flächenbereich, d. h. sowohl über den Flächenbereich des Koagulationsabschnittes 18b als auch über den Flächenbereich des Schneidabschnittes 18a, als Koagulationselektrode wirken, während der verjüngt ausgebildete Schneidabschnitt 18a allein für einen späteren Schneidvorgang zur Verfügung steht. Für den Schneidvorgang ist es vorgesehen, dass dem Schneidabschnitt 18a ein Schneidstrom zugeführt wird, der unterschiedlich dem Koagulationsstrom ist.

Ist der Schneidabschnitt 18a als Kante mit einem dreieckförmigen Querschnitt gem. Fig. 1 ausgebildet, so erlaubt der sanfte Übergang von einem großen Flächenbereich bis zur kantenförmigen Verjüngung in besonderem Maße, das gesamte Elektrodenteil 18 bei ausreichender Gewebedicke als Koagulationselektrode einzusetzen und in einem fortgeschrittenen Operationsstadium den Schneidabschnitt 18a allein zum Schneiden zu verwenden.

Ist der Schneidabschnitt 18a als Kante mit einem kreisförmigen Querschnitt an dem mindestens einen Elektrodenteil 18 ausgebildet, wie in Fig. 2 gezeigt, so steht eine relative große Elektrodenfläche für den Koagulationsvorgang zur Verfügung, während der als Kante ausgebildete Schneidabschnitt 18a bei genügend großer Gewebedicke kaum ins Gewicht fällt. Während des weiteren Operationsverlaufs, bei ausreichender Nähe gegenüberliegender Elektrodenteile 18, 19 des elektrochirurgischen Instruments 10 lässt sich aufgrund der kantenförmigen Ausgestaltung des Schneidabschnitts 18a die Stromdichte derart erhöhen, dass ein Schneidvorgang ermöglicht wird.

Bei einem gemäß Fig. 3 im Wesentlichen kugelförmig an dem mindestens einen Elektrodenteil 18 ausgebildeten Schneidabschnitt 18a lässt sich die Schneidefläche größer halten und ein entsprechend breiter Schnitt durchführen.

Im Übrigen kann der Schneidabschnitt 18a, 19a auch spitzen-, nadel- oder schlingenförmig ausgebildet sein.

Fig. 4 zeigt eine Elektrodenanordnung, bei der der Schneidabschnitt 18a, 19a jeweils an den sich gegenüberliegenden Elektrodenteilen 18, 19 ausgebildet ist. Damit sind auch hier an beiden Elektrodenteilen 18, 19 explizite Koagulationsabschnitte 18b, 19b vorgesehen. Auch diese Abbildung ist lediglich schematisch zu verstehen. Aufgrund der für das Schneiden ausgelegten Abschnitte 18a, 19a ist eine besonders präzise Schneidwirkung erreichbar, weil die Stromdichte an beiden Elektrodenteilen 18, 19 erhöht werden kann.

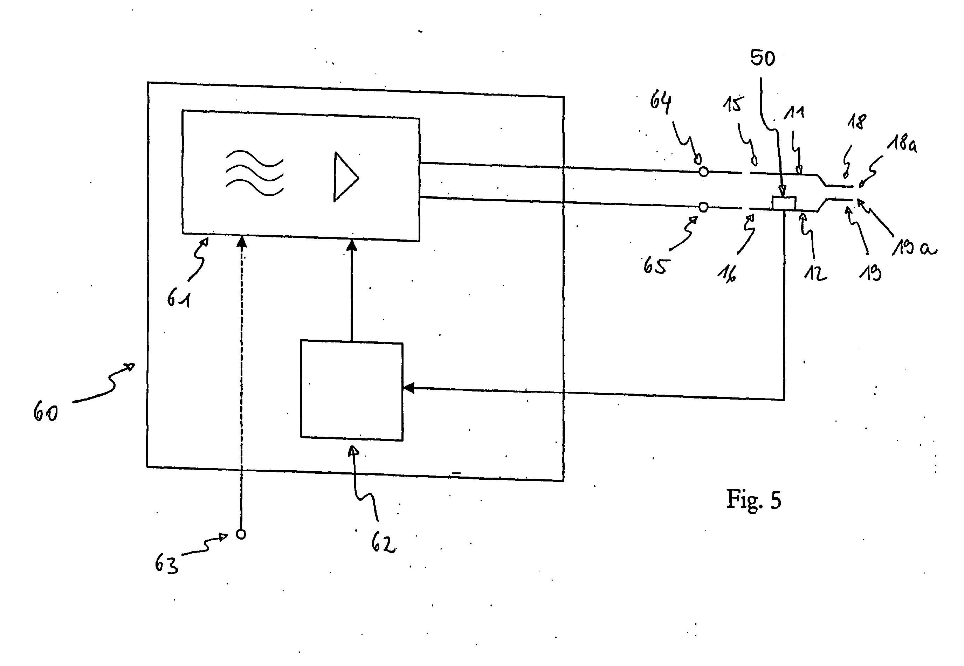

Fig. 5 zeigt ein funktionales Blockschaltbild, bei der das elektrochirurgische Instrument 10 mit einem Hochfrequenz-Chirurgiegerät 60 verbunden ist. Dabei sind ausschließlich und damit schematisch die für die Erläuterung der Erfindung wesentlichen Komponenten des HF-Chirurgiegerätes 60 gezeigt.

Das HF-Chirurgiegerät 60 weist einen Eingangsanschluss 63 beispielsweise zum Anschließen von Betätigungseinrichtungen, wie Finger- und/oder Fußschalter (nicht gezeigt), zum Aktivieren und/oder Deaktivieren des HF-Stromes auf. Die Betätigungseinrichtungen lassen sich hier vorzugsweise über eine Computeranordnung realisieren und sind in der praktischen Anwendung über eine Steuerungseinheit (nicht gezeigt) mit einem HF-Generator 61 verbunden. Der Einfachheit halber ist der Eingangsanschluss 63 in dieser Darstellung unmittelbar mit dem HF-Generator 61 verbunden und daher mit einer gestrichelten Linie dargestellt. Ausgangsseitig sind an dem HF-Chirurgiegerät 60 ein erster Ausgangsanschluss 64 und ein zweiter Ausgangsanschluss 65 vorgesehen, über die das elektrochirurgische Instrument 10 anschließbar ist.

Kernstück des HF-Chirurgiegerätes 60 ist der steuerbare HF-Generator 61 zum Erzeugen eines HF-Stromes, genauer gesagt, zum Erzeugen einer Spannung U_{HF-} Über die Einstellung der Spannung U_{HF} lassen sich die gewünschten Stromstärken I_{HF} für die verschiedenen Betriebsmodi, wie Koagulieren oder Schneiden, festlegen. Der HF-Generator 61 ist mit einer Steuerungseinheit 62 verbunden. Die Steuerungseinheit 62 ist ausgebildet, um Signale von Schalteinrichtungen 50 zu empfangen, die an dem elektrochirurgischen Instrument 10 angeordnet sind.

Die Schalteinrichtungen 50 sind zwischen Branchen 11, 12 des elektrochirurgischen Instruments angeordnet und erfassen einen Schwellenwert, z. B. als definierten Abstand zwischen den Branchen 11, 12, d.h. zwischen den Elektrodenteilen 18, 19. Der Abstand zwischen den Elektrodenteilen 18, 19 dient hier als Kennzeichen dafür, dass ein Schneidvorgang durchgeführt werden kann und ist dabei auf die Höhe der eingestellten HF-Spannung abgestimmt. Sobald ein bestimmter Abstand durch das Zusammenführen der Branchen 11, 12 erreicht ist, werden die Schalteinrichtungen 50 betätigt und leiten ein Signal an die Steuerungseinheit 62 weiter. Diese veranlasst dann die Zufuhr des entsprechenden Schneidstroms durch den HF-Generator 61 über proximale Enden 15, 16 des elektrochirurgischen Instruments 10 an die jeweiligen Schneidabschnitte 18a, 19a. Es sei darauf hingewiesen, dass der Schneidabschnitt auch hier nur an einem oder aber an beiden Elektrodenteilen 18, 19 ausgebildet sein kann. Für nachfolgende Erläuterungen wird angenommen, dass beide Elektrodenteile 18, 19 einen Schneidabschnitt 18a, 19a aufweisen.

Zur Erfassung des Abstandes und zur Aktivierung der den Schneidstrom steuernden Steuerungseinheit 62 lassen sich Tastschalter, aber auch berührungslose Schalter, wie z. B. Reedkontakt oder Näherungsschalter, einsetzen. Tastschalter erlauben eine besonders kostengünstige Ausgestaltung des elektrochirurgischen Instruments 10. Berührungslose Schalter arbeiten im Wesentlichen verschleißfrei und äußerst präzise.

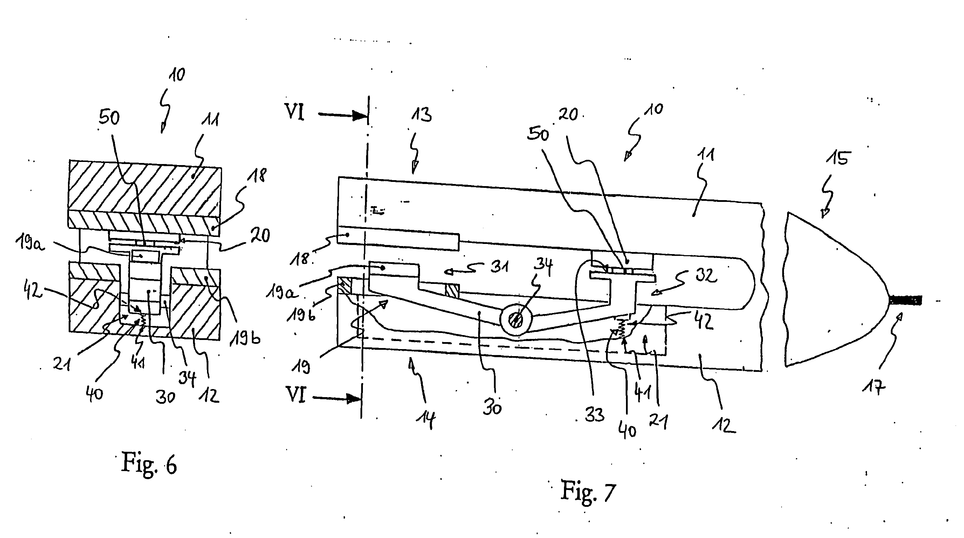

Für die genaue Anordnung der Schalteinrichtungen sei auf die Beschreibung der Fig. 6 und 7 verwiesen.

Die Erfassung des Schwellenwertes ist beispielsweise auch über eine der Steuerungseinheit 62 zugeordneten Einrichtung zur Widerstandsmessung (nicht gezeigt) möglich. Sobald in dem Gewebe aufgrund des Operationsverlaufes ein definierter Widerstand erreicht ist, veranlasst die Steuerungseinheit 62, dass dem jeweiligen Schneidabschnitt 18a, 19a der entsprechenden Schneidstrom zugeführt wird. Der Schwellenwert wird demgemäß als ohm'scher Widerstand des Gewebes erfasst.

Möglich ist es auch, der Steuerungseinheit 62 einen Lichtbogenmonitor und/oder einen Strommonitor (nicht gezeigt) zuzuordnen, die den Schwellenwert als optimalen Koagulationsendzeitpunkt erfassen. Der Schneidstrom wird dann mindestens dem jeweiligen Schneidabschnitt 18a 19a zugeführt, sobald die Koagulation aufgrund des von dem entsprechenden Monitor gelieferten Signals beendet wird. Damit erfolgt die Zuführung des Schneidstromes vorteilhafterweise zu einem für den Operationsverlauf idealen Zeitpunkt. Die Funktionsweisen des Strommonitors und Lichtbogenmonitors sind beispielsweise in der EP 0 253 012 B1 ausführlich beschrieben.

Fig. 6 zeigt eine Schnittansicht entlang der Linie VI - VI aus Fig. 7 einer Elektrodenanordnung in einer fünften Ausführungsform. Fig. 7 zeigt eine Seitenansicht des elektrochirurgischen Instruments 10 gemäß Fig. 6. Das elektrochirurgische Instrument 10 ist hier als pinzettenförmiges Instrument ausgeführt.

In den Fig. sind distale Enden 13, 14 der Branchen 11, 12 des elektrochirurgischen Instruments 10 abgebildet, sowie jeweilige Elektrodenteile 18, 19. Wie insbesondere Fig. 7 zu entnehmen, ist innerhalb einer Branche 12 ein zweiarmiger Hebel 30 mit einem ersten Ende 31 und einem zweiten Ende 32 über eine Drehachse 34 drehbar gelagert, wobei das erste Ende 31 zur Aufnahme des Schneidabschnittes 19a und das zweite Ende 32 zur Kontaktierung mit der gegenüberliegenden Branche 11 oder einem an der gegenüberliegenden Branche 11 vorgesehenen Abstandshalter 20 vorgesehen ist. Der Hebel 30 dient hier der Positionierung des Schneidabschnittes 19a, so dass dieser relativ zu dem für die Koagulation vorgesehenen Abschnitt 19b bewegbar ist. Das heißt bei dieser Ausführungsform ist das Elektrodenteil 19 aus zwei voneinander unabhängigen Abschnitten, dem Koagulationsabschnitt 19b und dem Schneidabschnitt 19a, ausgebildet.

Zur Aufnahme des Hebels 30 in die Branche 12 weist diese eine Ausnehmung 21 auf, in der der Hebel 30 an seinem ersten Ende 31 vorzugsweise vollständig versenkbar ist. Die Ausnehmung ist sowohl in dem Koagulationsabschnitt 19b der Branche 12 als auch in der Branche 12 selbst ausgebildet. Durch die Möglichkeit des Versenkens des Schneidabschnittes 19a wird eine Beeinträchtigung der Koagulationselektrode 19b während des Koagulierens durch den Schneidabschnitt 19a vermieden.

Bei Zusammenführung der Branchen 11, 12 durch den Operateur erfolgt eine stetige Annäherung des Abstandshalters 20 an das zweite Ende 32 des Hebels 30. Das zweite Ende 32 weist in dieser Ausführungsform eine Auflagefläche 33 auf. Sobald der Abstandshalter 20 an der gegenüberliegenden Branche 11 mit der Auflagefläche 33 in Berührung kommt, hebt sich das erste Ende 31 des Hebels 30 aus einer Ruhesposition aus der Branche 12 heraus, so dass der Schneidabschnitt 19a über die Koagulationselektrode 19b hinaussteht. Der Schneidabschnitt 19a und das gegenüberliegende Elektrodenteil 18 können nun in einer Schneidphase zusammenwirken. Der Abstandshalter 20 oder die Auflagefläche 33 weisen dazu die Schalteinrichtungen 50 auf, wie sie oben bereits ausführlich beschrieben wurden. Durch Kontaktierung des Abstandshalters 20 bzw. der Auflagefläche 33 mit den Schalteinrichtungen 50 lässt sich dann bei deren Betätigung der entsprechende Schneidstrom über Stromzuführungseinrichtungen 17, wie ebenfalls oben beschrieben, an den Schneidabschnitt 19a zuführen.

In der den Hebel 30 aufnehmenden Branche 12 ist ein Federelement 40 vorgesehen. Dieses ist an einem ersten Ende 41 mit der Branche 12 und an einem zweiten Ende 42 mit dem zweiten Hebelende 32 verbunden. Durch die Kontaktierung der Auflagefläche 33 des zweiten Hebelendes 32 mit dem Abstandshalter 20 der gegenüberliegenden Branche 11, bzw. mit den an dem Abstandshalter 20 oder an der Auflagefläche 33 angeordneten Schalteinrichtungen 50 wird die an dem zweiten Hebelende 32 angebrachte Feder 40, z. B. eine Spiralfeder, zusammengedrückt. Sobald die Kontaktierung unterbrochen wird, stellt die Feder 40 den Hebel 30 in seine Ruhesposition zurück, so dass das den Schneidabschnitt 19a aufweisende Hebelende 31 in der Branche 12 versenkt wird. Damit stünde die Koagulationselektrode 19b wieder für eine Koagulation zur Verfügung. Das Federelement 40 ist ein einfach einzubauendes und kostengünstiges Bauteil, das kaum verschleißanfällig stets die geforderte Funktion erfüllt.

Die Schalteinrichtungen 50 können im Übrigen auch an anderen Stellen der Branchen 11, 12 platziert werden. Hier würden sich dann insbesondere berührungslose Schalter anbieten, die auch ohne unmittelbare Kontaktierung ein Signal an die Steuerungseinheit bei Erreichen eines definierten Abstandes zwischen den Elektrodenteilen 18, 19 weiterleiten.

Das erste Ende des zweiarmigen Hebels 31 kann eine Aufnahmevorrichtung für den Schneidabschnitt 19a aufweisen, so dass dieser beispielsweise für Reinigungszwecke einfach von dem elektrochirurgischen Instrument entfernbar ist. Alternativ ist es möglich, das erste Hebelende 31 mit dem Schneidabschnitt 19a integral auszubilden. So ist eine äußerst kostengünstige Vorrichtung herstellbar.

Aufgrund der einfachen Mechanik der soeben beschriebenen Positionierungseinrichtung 30 für den Schneidabschnitt 19a ist auf einfache und kostengünstige Weise ein multifunktionales elektrochirurgisches Instrument 10 herstellbar.

Der Schneidabschnitt 18a, 19a lässt sich bevorzugt mit einer Anti-Haftbeschichtung und/oder mit einer Schicht aus abbrandfestem Material ausbilden. Damit lässt sich ein Anbrennen von Gewebe bzw. der Verschleiß des Schneidabschnittes vermeiden.

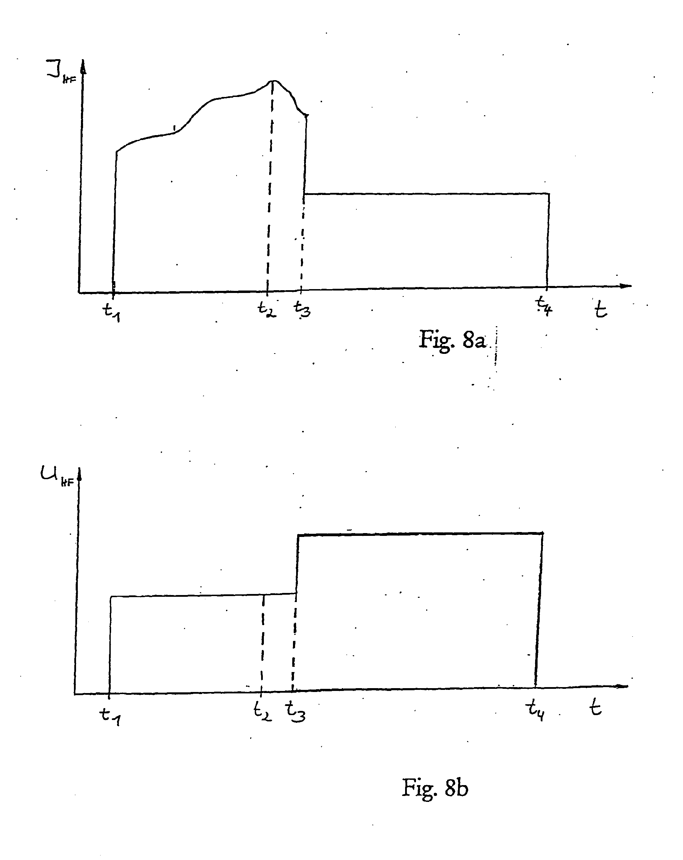

In Fig. 8a ist ein typischer Verlauf der Stromstärke des HF-Stromes in Abhängigkeit verschiedener Betriebsmodi gezeigt. Auf der Ordinatenachse ist die Stromstärke I_{HF}, auf der Abszissenachse ist die Zeit t aufgetragen. Fig. 8b zeigt das zu Fig. 8a gehörige Spannungs-Zeit-Diagramm. Auf der Ordinatenachse ist die Spannung U_{HF}, auf der Abszissenachse ist wiederum die Zeit t aufgetragen. Da es sich bei beiden Diagrammen um eine schematische Darstellung handelt, sind die Einheiten nicht aufgeführt.

Gemäß Fig. 8a wird zu einem Zeitpunkt t₁ ein Koagulationsmodus eingeschaltet, der Strom beginnt, durch das zu koagulierende Gewebe zu fließen. Aufgrund der Erwärmung des Gewebes steigt die Stromstärke I_{HF} bis zu einem Zeitpunkt t₂ an. Ab dem Zeitpunkt t₂ beginnt das Gewebe zu koagulieren, das heißt eine Dampfphase setzt ein. Aufgrund der durch den HF-Strom bedingten Wärmeentwicklung ist ein definierter Gewebebereich durch Eiweißkoagulation und Dehydratation veränderbar bzw. zerstörbar. Die im Solzustand vorliegenden kolloiden Gewebebestandteile gehen dabei zuerst in den Gelzustand über, wobei sich die nun gelförmigen Gewebebestandteile anschließend unter Austritt von Flüssigkeit weiter verdichten; das Gewebe verkocht. Der Widerstand des Gewebes steigt demgemäß an, so dass die Stromstärke I_{HF} aufgrund der sinkenden Leitfähigkeit des Gewebes bis zu einem Zeitpunkt t₃ abnimmt. Hat die Austrocknung des Gewebes ein bestimmtes Stadium erreicht, kommt die Koagulation zum Stillstand. Zwischen den Zeitpunkten t₃ und t₄ ist ein Schneidmodus aktiviert. Die Abbildung zeigt hier einen relativ konstanten Verlauf der Stromstärke I_{HF} aufgrund des im Wesentlichen gleichbleibenden Gewebewiderstandes während des Schneidvorgangs.

Gemäß Fig. 8b ist an dem HF-Generator für die Zeitspanne t₁ bis t₃ eine bestimmte Spannung UHF eingestellt. Der auf den Koagulationsmodus folgende Schneidmodus erfordert eine Erhöhung dieser Spannung UHF zwischen den Zeitpunkten t₃ und t₄. Die für das Schneiden benötigte Stromstärke IHF ist schließlich von der eingestellten Spannung UHF und von dem Gewebewiderstand des bereits koagulierten Gewebes abhängig. Zum Zeitpunkt t₄ lässt sich der Schneidmodus beispielsweise durch eine selbsttätige Abschaltvorrichtung beenden.

An dieser Stelle sei darauf hingewiesen, dass alle oben beschriebenen Teile für sich alleine gesehen und in jeder Kombination, insbesondere die in den Zeichnungen dargestellten Details als erfindungswesentlich beansprucht werden. Abänderungen hiervon sind dem Fachmann geläufig.

### Bezugszeichenliste

- 10: Elektrochirurgisches Instrument
- 11: Klemmenteil, Branche
- 12: Klemmenteil, Branche
- 13: Distales Ende
- 14: Distales Ende
- 15: Proximales Ende
- 16: Proximales Ende
- 17: Stromzuführungseinrichtungen
- 18: Elektrodenteil
- 18a: Schneidabschnitt, Schneidelektrode
- 18b: Koagulationsabschnitt, Koagulationselektrode
- 19: Elektrodenteil
- 19a: Schneidabschnitt, Schneidelektrode
- 19b: Koagulationsabschnitt, Koagulationselektrode
- 20: Abstandshalter
- 21: Ausnehmung

- 30: Zweiarmiger Hebel, Positionierungseinrichtung
- 31: Erstes Ende des Hebels
- 32: Zweites Ende des Hebels
- 33: Auflagefläche
- 34: Drehachse

- 40: Federelement
- 41: Erstes Ende des Federelements
- 42: Zweites Ende des Federelements

- 50: Schalteinrichtungen

- 60: HF-Chirurgiegerät
- 61: HF-Generator
- 62: Steuerungseinheit
- 63: Eingangsanschluss
- 64: Erster Ausgangsanschluss
- 65: Zweiter Ausgangsanschluss

## Patentansprüche

1. Elektrochirurgisches Instrument mit
- zwei gelenkig miteinander verbundenen Branchen (11, 12), die zum Öffnen oder Schließen entsprechend einem Klemm-, Spreiz- oder Schneidwerkzeug betätigbar sind,
- Elektrodenteile (18, 19) an distalen Enden (13, 14) der Branchen (11, 12) zum Fassen von Gewebe und zum Durchleiten eines Koagulationsstromes durch das Gewebe zu dessen Koagulation, die elektrisch voneinander isoliert sind,
- Stromzuführungseinrichtungen (17) zum Zuführen des Koagulationsstromes zu den Elektrodenteilen (18, 19),
- wobei mindestens an einem Elektrodenteil (18, 19) ein als Schneidelektrode ausgelegter Schneidabschnitt (18a, 19a) ausgebildet ist, so dass das Elektrodenteil (18, 19) den Schneidabschnitt (18a, 19a) und einen Koagulationsabschnitt (18b, 19b) aufweist,
wobei der Schneidabschnitt (18a, 19a) mittels Positionierungseinrichtungen (30) als relativ zu dem Koagulationsabschnitt (18b, 19b) bewegbarer Bestandteil an diesem ausgebildet ist,
- und wobei eine Steuerungseinheit (62) zur Steuerung des HF-Stromes derart vorgesehen ist, dass bei Erreichen eines Eigenschaften des erfassten Gewebes kennzeichnenden Schwellenwertes mindestens dem Schneidabschnitt (18a, 19a) ein Schneidstrom zugeführt wird, der unterschiedlich ist zum Koagulationsstrom,
**dadurch gekennzeichnet, dass**
die Positionierungseinrichtungen
- einen drehbar in einer der Branchen (12) gelagerten zweiarmigen Hebel (30) mit einem ersten Ende (31) und einem zweiten Ende (32) aufweisen, wobei das erste Ende (31) zur Aufnahme des Schneidabschnittes (19a) und das zweite Ende zur Kontaktierung mit der gegenüberliegenden Branche (11) oder einem an der gegenüberliegenden Branche (11) angeordneten Abstandshalter (20) vorgesehen ist, so dass bei Kontaktierung der Schneidabschnitt (19a) in Richtung des gegenüberliegenden Elektrodenteils (18) bewegbar ist,
- eine Rückstellvorrichtung (40) aufweisen, so dass der Schneidabschnitt (19a) nach Beendigung der Kontaktierung zurück in die Ausgangslage bewegbar ist.

2. Elektrochirurgisches Instrument nach Anspruch 1,
**dadurch gekennzeichnet, dass**
der Steuerungseinheit (62) Schalteinrichtungen (50) zugeordnet sind, die den Schwellenwert als definierten Abstand zwischen den Branchen (11, 12) erfassen, so dass der Schneidstrom in Abhängigkeit des Abstandes zugeführt wird.

3. Elektrochirurgisches Instrument nach Anspruch 2,
**dadurch gekennzeichnet, dass**
die Schalteinrichtungen (50) an mindestens einer der Branchen (11, 12) und/oder an einem mindestens an einer der Branchen (11, 12) angeordneten Abstandshalter (20) vorgesehen sind.

4. Elektrochirurgisches Instrument nach Anspruch 2 oder 3,
**dadurch gekennzeichnet, dass**
die Schalteinrichtungen (50) als ein Tastschalter ausgebildet sind.

5. Elektrochirurgisches Instrument nach einem der Ansprüche 2 bis 4,
**dadurch gekennzeichnet, dass**
die Schalteinrichtungen (50) als berührungslose Schalter ausgebildet sind.

6. Elektrochirurgisches Instrument nach Anspruch 5,
**dadurch gekennzeichnet, dass**
die berührungslosen Schalter als ein Näherungsschalter oder ein Reedkontakt ausgebildet sind.

7. Elektrochirurgisches Instrument nach einem der vorhergehenden Ansprüche,
**dadurch gekennzeichnet, dass**
der Steuerungseinheit (62) eine Einrichtung zur Widerstandsmessung zugeordnet ist, die den Schwellenwert als ohm'schen Widerstand des Gewebes erfasst, so dass der Schneidstrom in Abhängigkeit des ohm'schen Widerstandes zugeführt wird.

8. Elektrochirurgisches Instrument nach einem der vorhergehenden Ansprüche,
**dadurch gekennzeichnet, dass**
der Steuerungseinheit (62) ein Lichtbogenmonitor und/oder Strommonitor zugeordnet sind, die den Schwellenwert als optimalen Koagulationsendzeitpunkt erfassen, so dass der Schneidstrom in Abhängigkeit des Koagulationsendzeitpunktes zugeführt wird.

9. Elektrochirurgisches Instrument nach einem der vorhergehenden Ansprüche,
**dadurch gekennzeichnet, dass**
der Schneidabschnitt (18a, 19a) an dem mindestens einen Elektrodenteil (18, 19) als ein in Bezug auf den Koagulationsabschnitt (18b, 19b) des mindestens einen Elektrodenteils (18, 19) verjüngter Bereich ausgebildet ist.

10. Elektrochirurgisches Instrument nach einem der vorhergehenden Ansprüche,
**dadurch gekennzeichnet, dass**
der Schneidabschnitt (18a, 19a) als Kante mit einem im Wesentlichen dreieckförmigen Querschnitt an dem mindestens einen Elektrodenteil (18, 19) ausgebildet ist.

11. Elektrochirurgisches Instrument nach einem der Ansprüche 1 bis 9,
**dadurch gekennzeichnet, dass**
der Schneidabschnitt (18a, 19a) als Kante mit einem im Wesentlichen kreisförmigen Querschnitt an dem mindestens einen Elektrodenteil (18, 19) ausgebildet ist.

12. Elektrochirurgisches Instrument nach einem der Ansprüche 1 bis 9,
**dadurch gekennzeichnet, dass**
der Schneidabschnitt (18a, 19a) im Wesentlichen kugelförmig an dem mindestens einen Elektrodenteil (18, 19) ausgebildet ist.

13. Elektrochirurgisches Instrument nach einem der Ansprüche 1 bis 9,
**dadurch gekennzeichnet, dass**
der Schneidabschnitt (18a, 19a) spitzen-, nadel- oder schlingenförmig ausgebildet ist.

14. Elektrochirurgisches Instrument nach einem der vorhergehenden Ansprüche,
**dadurch gekennzeichnet, dass**
der Schneidabschnitt jeweils an sich gegenüberliegenden Elektrodenteilen (18, 19) (18a, 19a) ausgebildet ist.

15. Elektrochirurgisches Instrument nach einem der vorhergehenden Ansprüche,
**dadurch gekennzeichnet, dass**
am ersten Ende (31) des Hebels (30) eine Aufnahmevorrichtung für den Schneidabschnitt (19a) angebracht ist.

16. Elektrochirurgisches Instrument nach einem der vorhergehenden Ansprüche,
**dadurch gekennzeichnet, dass**
der Schneidabschnitt (19a) als integraler Bestandteil des ersten Endes (31) des Hebels (30) ausgebildet ist.

17. Elektrochirurgisches Instrument nach einem der vorhergehenden Ansprüche,
**dadurch gekennzeichnet, dass**
die Rückstellvorrichtung (40) als in der die Positionierungseinrichtungen (30) aufweisenden Branche (12) angeordnetes Federelement vorgesehen ist.

18. Elektrochirurgisches Instrument nach einem der vorhergehenden Ansprüche,
**dadurch gekennzeichnet, dass**
der Schneidabschnitt (18a, 19a) aus einer Anti-Haftbeschichtung und/oder aus abbrandfestem Material ausgebildet ist.

## Claims

1. Electrosurgical instrument, comprising
- two branches (11, 12) joined in articulated manner to one another, which can be actuated to open or close, respectively, a clamping, spreading or cutting tool,
- electrode parts (18, 19) at distal ends (13, 14) of the branches (11, 12) for grasping tissue and for conducting a coagulating current through the tissue in order to coagulate said tissue, said electrode parts being electrically insulated from one another,
- current feed devices (17) for feeding the coagulating current to the electrode parts (18, 19),
- wherein at least at one electrode part (18, 19), a cutting portion (18a, 19a) configured as a cutting electrode is provided so that the electrode part (18, 19) comprises the cutting portion (18a, 19a) and a coagulating portion (18b, 19b), wherein the cutting portion (18a, 19a) is provided as a component of the coagulating portion (18b, 19b) and movable relative thereto by means of positioning devices,
- and wherein a control unit (62) for controlling the HF current is provided such that, on reaching a threshold value characterising a property of the grasped tissue, a cutting current which is different from the coagulating current is fed at least to the cutting portion (18a, 19a),
**characterised in that**
the positioning devices
- comprise a two-armed lever (30) pivotably mounted in one of the branches (12), having a first end (31) and a second end (32), wherein the first end (31) is provided for receiving the cutting portion (19a) and the second end is provided for contacting the opposing branch (11) or a spacer (20) arranged on the opposing branch (11), so that during contacting, the cutting portion (19a) is movable in the direction of the opposing electrode part (18),
- a return apparatus (40), so that the cutting portion (19a) is movable back into the starting position after completion of the contacting.

2. Electrosurgical instrument according to claim 1, **characterised in that** switching devices (50) are associated with the control unit (62) , said switching devices detecting the threshold value as a defined distance between the branches (11, 12), so that the cutting current is supplied depending on the distance.

3. Electrosurgical instrument according to claim 2, **characterised in that** the switching devices (50) are provided on at least one of the branches (11, 12) and/or on a spacer (20) arranged on at least one of the branches (11, 12).

4. Electrosurgical instrument according to claim 2 or 3, **characterised in that** the switching devices (50) are configured as a touch contact switch.

5. Electrosurgical instrument according to one of the claims 2 to 4, **characterised in that** the switching devices (50) are configured as contact-free switches.

6. Electrosurgical instrument according to claim 5, **characterised in that** the contact-free switches are configured as a proximity switch or a reed contact.

7. Electrosurgical instrument according to one of the preceding claims, **characterised in that** the control unit (62) is associated with a device for resistance measurement which detects the threshold value as an ohmic resistance of the tissue, so that the cutting current is fed depending on the ohmic resistance.

8. Electrosurgical instrument according to one of the preceding claims, **characterised in that**, associated with the control unit (62) is an arc monitor and/or a current monitor, which detect the threshold value as an optimum coagulation end time point, so that the cutting current is fed depending on the coagulation end time point.

9. Electrosurgical instrument according to one of the preceding claims, **characterised in that** the cutting portion (18a, 19a) is provided on the at least one electrode part (18, 19) as a region which is narrowed relative to the coagulating portion (18b, 19b) of the at least one electrode part (18, 19).

10. Electrosurgical instrument according to one of the preceding claims, **characterised in that** the cutting portion (18a, 19a) is configured as an edge with an essentially triangular cross-section on the at least one electrode part (18, 19).

11. Electrosurgical instrument according to one of the claims 1 to 9, **characterised in that** the cutting portion (18a, 19a) is configured as an edge with an essentially circular cross-section on the at least one electrode part (18, 19).

12. Electrosurgical instrument according to one of the claims 1 to 9, **characterised in that** the cutting portion (18a, 19a) is configured essentially spherical on the at least one electrode part (18, 19).

13. Electrosurgical instrument according to one of the claims 1 to 9, **characterised in that** the cutting portion (18a, 19a) is configured pointed, needle-shaped or noose-shaped.

14. Electrosurgical instrument according to one of the preceding claims, **characterised in that** the cutting portions (18a, 19a) is provided on mutually opposing electrode parts (18, 19).

15. Electrosurgical instrument according to one of the preceding claims, **characterised in that** a receptacle device for the cutting portion (19a) is mounted on the first end (31) of the lever (30).

16. Electrosurgical instrument according to one of the preceding claims, **characterised in that** the cutting portion (19a) is configured as an integral component of the first end (31) of the lever (30).

17. Electrosurgical instrument according to one of the preceding claims, **characterised in that** the return apparatus (40) is provided as a spring element arranged in the branch (12) comprising the positioning devices (30).

18. Electrosurgical instrument according to one of the preceding claims, **characterised in that** the cutting portion (18a, 19a) is made from an anti-adhesion coating and/or a heat corrosion-resistant material.

## Revendications

1. Instrument d'électrochirurgie comportant
- deux branches (11, 12) reliées entre elles de manière articulée et actionnables en ouverture ou fermeture à la manière d'un instrument de serrage, d'écartement ou de coupe,
- des parties électrodes (18, 19) agencées aux extrémités distales (13, 14) des branches (11, 12) pour saisir un tissu et y faire passer un courant de coagulation en vue de coaguler ledit tissu, lesdites parties électrodes étant isolées électriquement l'une de l'autre,
- des dispositifs d'alimentation électrique (17) pour amener le courant de coagulation vers les parties électrodes (18, 19),
- une zone de coupe (18a, 19a) conçue sous la forme d'une électrode de coupe étant formée sur au moins une partie électrode (18, 19), de sorte que la partie électrode (18, 19) présente la zone de coupe (18a, 19a) et une zone de coagulation (18b, 19b),
la zone de coupe (18a, 19a) étant formée sur la zone de coagulation (18b, 19b) en tant que composant mobile par rapport à ladite zone de coagulation grâce à des dispositifs de positionnement (30),
- et une unité de commande (62) du courant HF étant prévue de façon que lorsqu'une valeur seuil caractéristique d'une propriété du tissu saisi est atteinte, la zone de coupe (18a, 19a) au moins soit alimentée par un courant de coupe différent du courant de coagulation,
**caractérisé en ce que**
les dispositifs de positionnement
- présentent un levier (30) à deux bras monté rotatif dans une des branches (12), avec une première extrémité (31) et une seconde extrémité (32), la première extrémité (31) étant prévue pour recevoir la zone de coupe (19a) et la seconde extrémité pour mettre en contact avec la branche opposée (11) ou avec un dispositif d'espacement (20) agencé sur la branche opposée (11) de sorte que, lors de la mise en contact, la zone de coupe (19a) est mobile en direction de la partie électrode opposée (18),
- présentent un dispositif de rappel (40) de façon que la zone de coupe (19a) puisse retourner à la position de repos après la mise en contact.

2. Instrument d'électrochirurgie selon la revendication 1, **caractérisé en ce que**
à l'unité de commande (62) sont associés des dispositifs de connexion (50) qui détectent la valeur seuil en tant que distance définie entre les branches (11, 12), de sorte que le courant de coupe est envoyé en fonction de la distance.

3. Instrument d'électrochirurgie selon la revendication 2,
**caractérisé en ce que**
les dispositifs de connexion (50) sont prévus sur au moins une des branches (11, 12) et/ou sur un dispositif d'espacement (20) agencé sur au moins une des branches (11, 12).

4. Instrument d'électrochirurgie selon la revendication 2 ou la revendication 3,
**caractérisé en ce que**
les dispositifs de connexion (50) sont conçus sous la forme d'un bouton-poussoir.

5. Instrument d'électrochirurgie selon l'une quelconque des revendications 2 à 4,
**caractérisé en ce que**
les dispositifs de connexion (50) sont conçus sous la forme de commutateurs sans contact.

6. Instrument d'électrochirurgie selon la revendication 5,
**caractérisé en ce que**
les commutateurs sans contact sont conçus sous la forme d'un détecteur de proximité ou d'un contact reed.

7. Instrument d'électrochirurgie selon l'une quelconque des revendications précédentes,
**caractérisé en ce que**
à l'unité de commande (62) est associé un dispositif de mesure de résistance, qui détecte la valeur seuil en tant que résistance ohmique du tissu, de sorte que le courant de coupe est envoyé en fonction de la résistance ohmique.

8. Instrument d'électrochirurgie selon l'une quelconque des revendications précédentes,
**caractérisé en ce que**
à l'unité de commande (62) est associé un moniteur d'arc électrique et/ou un moniteur de courant qui détecte(nt) la valeur seuil en tant qu'instant de fin de coagulation optimal, de sorte que le courant de coupe est envoyé en fonction de l'instant de fin de coagulation.

9. Instrument d'électrochirurgie selon l'une quelconque des revendications précédentes,
**caractérisé en ce que**
la zone de coupe (18a, 19a) est formée sur la au moins une partie électrode (18, 19) sous la forme d'un rétrécissement par rapport à la zone de coagulation (18b, 19b) de la au moins une partie électrode (18, 19).

10. Instrument d'électrochirurgie selon l'une quelconque des revendications précédentes,
**caractérisé en ce que**
la zone de coupe (18a, 19a) est formée sur la au moins une partie électrode (18, 19) sous la forme d'une arête de section transversale sensiblement triangulaire.

11. Instrument d'électrochirurgie selon l'une quelconque des revendications 1 à 9,
**caractérisé en ce que**
la zone de coupe (18a, 19a) est formée sur la au moins une partie électrode (18, 19) sous la forme d'une arête de section transversale sensiblement circulaire.

12. Instrument d'électrochirurgie selon l'une quelconque des revendication 1 à 9,
**caractérisé en ce que**
la zone de coupe (18a, 19a) est formée sur la au moins une partie électrode (18, 19) sous une forme sensiblement sphérique.

13. Instrument d'électrochirurgie selon l'une quelconque des revendications 1 à 9,
**caractérisé en ce que**
la zone de coupe (18a, 19a) est pointue ou en forme d'aiguille ou de boucle.

14. Instrument d'électrochirurgie selon l'une quelconque des revendications précédentes,
**caractérisé en ce que**
la zone de coupe est formée sur des parties électrodes en regard (18, 19) (18a, 19a).

15. Instrument d'électrochirurgie selon l'une quelconque des revendications précédentes,
**caractérisé en ce que**
un logement de la zone de coupe (19a) est établi sur la première extrémité (31) du levier (30).

16. Instrument d'électrochirurgie selon l'une quelconque des revendications précédentes,
**caractérisé en ce que**
la zone de coupe (19a) est conçue comme faisant partie intégrante de la première extrémité (31) du levier (30).

17. Instrument d'électrochirurgie selon l'une quelconque des revendications précédentes,
**caractérisé en ce que**
le dispositif de rappel (40) est prévu sous la forme d'un ressort agencé dans la branche (12) présentant les dispositifs de positionnement (30).

18. Instrument d'électrochirurgie selon l'une quelconque des revendications précédentes,
**caractérisé en ce que**
la zone de coupe (18a, 19a) est composée d'un revêtement antiadhésif et/ou d'un matériau résistant à la brûlure.
